Europäisches Patentamt

European Patent Office

Office européen des brevets

(11) Veröffentlichungsnummer: **0 456 197 A1**

# EUROPÄISCHE PATENTANMELDUNG

(21) Anmeldenummer: 91107423.5

(22) Anmeldetag: 07.05.91

(51) Int. Cl.5: **C07K 7/08**, C07K 7/10,
A61K 37/02, A61K 39/42,
G01N 33/569

(30) Priorität: 10.05.90 DE 4015044

(43) Veröffentlichungstag der Anmeldung:
13.11.91 Patentblatt 91/46

(84) Benannte Vertragsstaaten:
AT BE CH DE DK ES FR GB GR IT LI LU NL SE

(71) Anmelder: BEHRINGWERKE
Aktiengesellschaft
Postfach 1140
W-3550 Marburg 1(DE)

(72) Erfinder: Bleul, Conrad
Römerstrasse 15a
W-6900 Heidelberg(DE)
Erfinder: Gissmann, Lutz, Prof. Dr.
Im Pirolweg 1
W-6908 Wiesloch(DE)
Erfinder: Müller, Martin
Husarenstrasse 14
W-6900 Heidelberg(DE)

(74) Vertreter: Becker, Heinrich Karl Engelbert, Dr.
et al
HOECHST AKTIENGESELLSCHAFT Central
Patent Department P.O. Box 80 03 20
W-6230 Frankfurt am Main 80(DE)

(54) Seroreaktive Epitope auf Proteinen des menschlichen Papillomavirus (HPV) 18.

(57) Die Erfindung betrifft seroreaktive Epitope auf Proteinen des menschlichen Papillomavirus HPV18.

Außerdem betrifft die Erfindung Peptide, die Aminosäuresequenzen besitzen, die ganz oder teilweise mit den Sequenzen der seroreaktiven Epitope übereinstimmen und Impfstoffe, die solche Peptide enthalten.

EP 0 456 197 A1

Die Erfindung betrifft Seroreaktive Regionen auf den Proteinen E1, E6 und E7 des menschlichen Papillomavirus (HPV) 18.

Weiterhin betrifft die Erfindung Impfstoffe, die Peptide enthalten, welche Aminosäuresequenzen der seroreaktiven Regionen der genannten Virusproteine umfassen und diagnostische Kits, welche die genannten Peptide enthalten.

HPV18 ist ein spezieller Typ des menschlichen Papillomavirus, der das erste Mal in Proc. Natl. Acad. Sci., USA 80, 3813-3815 (1983) beschrieben wurde.

Die DNA-Sequenz und die Organisation des viralen Genoms von HPV18 wurde in Virology 145, 181-185 (1985) publiziert.

HPV18 induziert nicht nur gutartige Schädigungen des Anogenitaltrakts, sondern auch maligne Tumoren des Uterushalses, des Penis und der Scheide. Zudem findet sich HPV18 jedoch in genitalen Ausschabungen von klinisch symptomlosen Individuen. Bis heute ist über die Immunantwort, die auf eine Infektion durch HPV18 und andere Papillomaviren erfolgt, wenig bekannt.

In ersten Experimenten wurden menschliche Seren von STD-Patienten, von Patienten, die an zervikalen Tumoren leiden und von gesunden Individuen auf die Anwesenheit von Antikörpern, die gegen virale Proteine gerichtet sind, getestet. Diese viralen Proteine wurden als Fusionsproteine, die an verschiedene prokaryotische Peptide über ihren N-Terminus kovalent gebunden waren, exprimiert. Solche Fusionsproteine wurden dann als Antigene in Western-Blot-Experimenten verwendet. Dieser Test ist jedoch relativ langwierig und kompliziert und nur mit großem Aufwand durchzuführen, so daß er nicht für eine quantitative Analyse von großen Mengen menschlichen Serums geeignet erscheint. Außerdem ist dieser Test nicht sehr spezifisch, weil die verschiedenen Papillomavirus-Typen auch im Hinblick auf ihr Proteincore verwandt sind und dadurch eine Kreuzreaktion von Antikörpern mit Proteinen bzw. Fusionsproteinen verschiedener Papillomavirus-Typen nicht ausgeschlossen werden kann.

Die Aufgabe der vorliegenden Erfindung ist deshalb die Identifizierung von viralen Strukturen des HPV18, die als Hilfsmittel in der Prophylaxe, der Diagnose und der Therapie von HPV18-induzierten Krankheiten des Menschen geeignet sind. Das Wissen um solche Strukturen (Proteindomänen) ist eine Voraussetzung für die Etablierung eines Tests mit dem große Mengen menschlichen Blutserums auf Anwesenheit von spezifischen HPV geprüft werden können.

Die vorliegende Erfindung umfaßt insofern ein serorekatives Epitop auf dem E1-Protein von HPV18 mit der folgenden Aminosäuresequenz

**TENSPLGERLEVDTELSPRLQEISLNS**

sowie seroreaktive Epitope auf dem E6-Protein von HPV18 mit einer der folgenden Aminosäuresequenzen

I.     **DPTRRPYKLPDLCTELNTSLQDIEITCVYCKT**

II.     **MARFEDPTRRPYKL**

III.     **AACHKCIDFYSRIRELRHYSDSVYGDTLEKLT**

sowie seroreaktive Epitope auf dem E7-Protein von HPV18 mit einer der folgenden Aminosäuresequenzen

I.     **VLHLEPQNEIPVDLLCHEQLSDSEEENDEIDGVNHQHLPARRAEPQRH und**

II.     **IDGVNHQHLPARR.**

Weiterhin umfaßt die Erfindung Peptide, die entweder eine oder mehrere erfindungsgemäße Aminosäuresequenz(en) der oben genannten seroreaktiven Epitope enthalten.

Die Erfindung umfaßt auch Impfstoffe, die auf Peptiden basierten, die eine oder mehrere Aminosäuresequenz(en) der oben genannten seroreaktiven Epitope der Proteine von HPV18 enthalten.

Spezifische Antikörper gegen HPV18 E1-, E6- und E7-Proteine können mit Hilfe eines erfindungsgemäßen diagnostischen Kits in Patientenseren nachgewiesen werden. Dieser Kit enthält die erfindungsgemäßen Peptide.

Im Sinne einer Prophylaxe können auch die spezifischen viralen Proteine, die die seroreaktiven Regionen enthalten, auch frühzeitig durch polyklonale Antikörper bzw. monoklonale Antikörper, die gegen diese Regionen gerichtet sind, im Blutserum identifiziert werden. Dementsprechend umfaßt diese Erfindung auch einen diagnostischen Kit, der polyklonale oder monoklonale Antikörper enthält, die spezifisch gegen die seroreaktiven Regionen des HPV18 gerichtet sind.

Zur Identifizierung der seroreaktiven Epitope wurden folgende voneinander unabhängige Methoden verwendet:

A. Ein Screening einer "Shot Gun"-Expressionsbank: Die im Bakterienplasmidvektor pSP65 klonierte HPV18-DNA wurde mit Ultraschallscherung und anschließender DNase-Behandlung auf eine durchschnittliche Fragmentgröße von 100 Basenpaaren gebracht. Die Enden dieser Fragmente wurden mit T4 DNA-Polymerase aufgefüllt und in den Phagenexpressionsvektor fuse 1 einligiert. Fuse 1 ist von dem Bakteriophagen fd abgeleitet und in Science 228, 1315-1317 (1985) beschrieben. Die Phagen wurden mit Escherichia coli K91 ausplattiert, Replika auf Nitrocellulosefilter abgezogen und die Filter mit geeigneten polyklonalen Kaninchenseren inkubiert. Positive Klone wurden in mehreren Vereinzelungsschritten isoliert und die immunreaktiven Peptidsequenzen durch DNA-Sequenzierung identifiziert.

B. Peptidüberlappung

127 überlappende Peptide, die kurzen Stücken der HPV18E6- und -E7-Proteine entsprechen, wurden an Polyethylen-"pins" synthetisiert, wobei die Fmoc-Chemie verwendet wurde (Proc. Natl. Acad. Sci., 82, 178 (1985)). Die Proteinsequenz der E6- und E7-Proteine wurde in 10mere unterteilt, die in 8 Aminosäuren mit dem nächsten Peptid übereinstimmen. Die Peptide wurden in den entsprechenden Antiseren durch ELISA ausgetestet.

Beispiel 1

Derivate des filamentösen Phasgen fd wurden benutzt um ein Expressionssystem für HPV18 DNA-Fragmente zu erhalten. Dazu wurde fuse 1 (fd-tet-J6, Science 228, 1315-1317 (1985); Gene 73, 305-318 (1988)) an der einzigen Pvull-Schnittstelle geschnitten. Genomische HPV18 DNA-Fragmente aus einem DNasel-Verdau nach DNA-Repair wurden mit einer T4-DNA-Ligase blunt-end einligiert. Zur Transformation des fuse 1-Vektors wurde der E. coli Stamm K802 (FgalK2 galT22 metB1 supE44 hsdr(2); Journal of Molecular Biology 16, 118-133 (1966)) nach dem Verfahren von Hanahan aus Journal of Molecular Biology 166, 557-580 (1988) verwendet. Die tet-resistenten Kolonien produzieren Bacteriophagen, die für die Bakterien wegen ihres F-Phänotyps nicht infektiös sind. Um die Phagen auszuplattieren wurde der E. coli Stamm K91 (F$^+$, ein Derivat von E. coli K38, Virology 49, 45-60 (1972)) verwendet.

Beispiel 2

Ungefähr 50 000 rekombinierte Phagen aus der oben beschriebenen Random-Bank wurden mit 0,2 ml exponentiell wachsenden E. coli K91-Zellen in 3,5 ml 0,5 % Agarose, die 10 mM MgSO$_4$ enthielt, auf Minimalagarplatten ausplattiert. Replika der Platten wurden auf Nitrocellulosefilter abgezogen und dann 6 h lang bei 37 $^\circ$C weiter auf frischem Minimalagar inkubiert, um das Signal zu verstärken. Danach wurden die Filter 60 min lang mit 10 % fettarmer Milch in PBS geblockt und über Nacht in 5 % Milch-PBS mit einer Verdünnung von HPV-spezifischen Antisera von 1:100 bis 1:1000 inkubiert. Statt der spezifischen HPV-Antisera können auch monoklonale Antikörper verwendet werden. Die Antisera wurden mit beschallten K91-Zellen präadsorbiert. Die Filter wurden dann 5x gewaschen und zwar 5 min lang in einer PBS/0,1 % Tween 20 und dann 3 h lang bei Raumtemperatur mit Ziegen-anti-Kaninchen-Antikörper oder, im Fall der Verwendung monoklonaler Antikörper, anti-Maus-Peroxidase-Antikörper (1:1000) in 5% fettarmer Milch inkubiert. Nach Waschen der Filter wurden sie in 50 ml PBS, das 30 mg Diaminobenzidin, 30 μl %iges H$_2$O$_2$ und 1,5 ml 1 %iges NiSO$_4$ enthielt, gefärbt. Danach wurden die Filter 30 min lang in H$_2$O gewaschen und danach auf Filterpapier getrocknet.

Mit dem polyklonalen Kaninchenserum gegen HPV18 E7 wurden primär 25 Phagen isoliert, von denen sich 18 in den weiteren Aufreinigungsschritten als positiv erwiesen. Anschließend wurden Phagenpartikel in Kultur angezüchtet und Einzelstrang-DNA präpariert.

Beispiel 3

Derselbe Ansatz wie in Beispiel 2 wurde auch für HPV18 E6-Protein gewählt. Da das verwendete polyklonale Kaninchenserum Kreuzreaktionen mit nichtviralen Epitopen aufwies, wurde neben der Western-Blot-methode mit spezifischen DNA-Fragmenten geprobt, um unter allen reaktiven Rekombinanten solche mit HPV18 E6-Anteilen zu identifizieren. Aus 70 000 rekombinanten Phagen wurden 15 isoliert und schließlich sequenziert. Das Epitop HPV18 E6 Nr. 1 wurde so zum Beispiel in der untersuchten Phagenbank insgesamt 10 mal gefunden.

Beispiel 4

Präparation von Einzelstrang-DNA aus fuse 1-Rekombinanten

Hierzu wurde eine Vorschrift aus Proc. Natl. Acad. Sci., USA 74, 5463-5467 (1977) verwendet. 50 ml LM wurden mit tet-resistenten E. coli K91-Zellen inkubiert, die das fuse 1-Plasmid trugen und dieser Ansatz wurde 16 h lang bei 37 °C inkubiert. Die Bakterien wurden dann bei 6000 rpm 30 min lang pelletiert. Nach dem Hinzufügen von 2 ml 40 %igem PEG 6000 und 2 mml 5 M Natriumacetat, pH 6,5 zum Überstand wurden die Phagen bei 0 °C 60 min lang präzipitiert und das Präzipitat bei 6000 rpm 60 min lang zentrifugiert. Das Pellet wurde in 0,3 ml TE resuspendiert. Nach zwei Extraktionen mit Phenol wurde die DNA präzipitiert. Ungefähr 25 % der Präparationen wurden dann zur Sequenzierung verwendet.

Beispiel 5

Sequenzierung

Zur DNA-Sequenzierung wurde die Standard-USB (United States Biochemicals)-Methode (USB, 1987) verwendet. Der universale Primer wurde durch ein 20mer Oligonucleotid (5'-TCCAGACGTTAGTAAATGAA-3') ersetzt.

Beispiel 6

Peptid-Synthese

127 überlappende Peptide, die in kurzen Stücken die ORFs HPV18 E6 und -E7 darstellen, wurden nach der Fmoc-Chemie an Polyethylen-"pins" synthetisiert, wie in Proc. Natl. Acad. Sci. 32, 178 (1985) und Proc. Natl. Acad. Sci. 81, 3998 (1985) beschrieben. Die Polyethylen-"pins", die mit $\beta$-Alanin derivatisiert wurden, wurden von CRB England erhalten. Abweichend von oben zitierten Publikationen wurde die Proteinsequenz in Dekapeptide eingeteilt, die mit dem Nachbarpeptid um 8 Aminosäuren überlappen. Die Synthese wurde ausgeführt mit Hilfe der Fmoc-Chemie und in situ-Aktivierung durch BOP (Castro's Reagens) (Tetrahedron Letters, 14, 1219 (1975)). Fmoc-Aminosäurederivate (6 $\mu$mol), BOP und N-Methylmorpholin-Lösung wurden in Polyethyleneinsätze (CRB) entsprechend der Peptidsequenz, die synthetisiert werden soll, verteilt. Alle anderen Reaktionen wurden nach dem CRB-Protokoll ausgeführt.

Beispiel 7

Die Polyethylen-"pins" wurden nach dem ELISA-Testverfahren mit den oben genannten polyklonalen Kaninchenseren inkubiert, gebundene Antikörper mit Protein-A-Peroxidase nachgewiesen. Ein durch unspezifische Bindungen entstehender Background wurde durch Protein-A-Inkubation ohne ersten Antikörper quantifiziert. Die reaktiven Peptide liegen in Bereichen, die durch das Phagenscreening als seroreaktives Epitop identifiziert wurden.

Alle Tests wurden an den Peptiden, die kovalent an die Polyethylen-"pins" gebunden vorliegen und an die sie ursprünglich auch synthetisiert wurden, durchgeführt. Racks mit 96 pins, die in einer solchen Konfiguration fixiert waren, daß sie in die Löcher von Miktrotiter-Platten eingebracht werden konnten, wurden benutzt. Die Inkubation für den ELISA wurde durchgeführt, während die pins in die Löcher hineingetaucht wurden. Die pins wurden mit Methanol und PBS gewaschen und danach mit 0,25 % Gelatine, 0,1 % Tween 20 in PBS 2 h lang bei 37 °C geblockt, gefolgt von einer 1 h Inkubation bei 37 °C mit Seren, die 1:200 bis 1:4000 in 0,125 % Gelatine und 0,05 % Tween 20 verdünnt wurden. Nach einem weiteren Waschschritt mit PBS/0,1 % Tween 20 wurden die pins 1 h lang bei 37 °C mit Protein-A-Peroxidase 1:4000 inkubiert, gefolgt von einem weiteren Waschschritt und Färben mit Tetramethylbenzidin (TMB) 15 min lang. Der Färbeschritt wurde durch Herausziehen der pins aus der Färbelösung und Hinzufügen von 100 $\mu$l einer 0,2 molaren $H_2SO_4$-Lösung beendet. Die Absorption wurde dann an einem automatischen ELISA-Reader gemessen. Um den Antikörper-Enzym-Komplex nach dem ELISA-Verfahren zu entfernen, wurden die pins für 1 h mit Ultraschall (Wasserbad, 30 W, 48 kHz) bei 60 °C in PBS/1 % SDS/0,1 % $\beta$-Mercaptoethanol inkubiert und wurden schließlich mit Methanol gewaschen. Die Effektivität dieser Prozedur wurde mit Hilfe von ELISA unter Verwendung von Protein A/Peroxidase ohne Beteiligung eines primären Serums ausgetestet. Die gleichen Peptide wurden mehr als 40 mal in den folgenden ELISA's getestet.

## Tabelle

### Seroreaktives Epitop E1 (HPV18)

bpl1193-TENSPLGERLEVDTELSPRLQEISLNS-bp1273

---

### Seroreaktives Epitop E6 (HPV18)

pb120-DPTRRPYKLPDLCTELNTSLQDIEITCVYCKT-bp215

MARFEDPTRRPYKL

bp294-AACHKCIDFYSRIRELRHYSDSVYGDTLEKLT-bp386

---

### Seroreaktives Epitop E7 (HPV18)

bp623-VLHLEPQNEIPVDLLCHEQLSDSEEENDEIDGVNHQHLPARRAEPQRH-bp763

IDGVNHQHLPARR

### Patentansprüche

1. Seroreaktives Epitop auf dem E1-Protein von HPV18 mit der folgenden Aminosäuresequenz

TENSPLGERLEVDTELSPRLQEISLNS

2. Seroreaktive Epitope auf dem E6-Protein von HPV18 mit einer der folgenden Aminosäuresequenzen

    I.    DPTRRPYKLPDLCTELNTSLQDIEITCVYCKT

    II.    MARFEDPTRRPYKL

    III.    AACHKCIDFYSRIRELRHYSDSVYGDTLEKLT

3. Seroreaktive Epitope auf dem E7-Protein von HPV18 mit einer der folgenden Aminosäuresequenzen

    I.    VLHLEPQNEIPVDLLCHEQLSDSEEENDEIDGVNHQHLPARRAEPQRH

    II.    IDGVNHQHLPARR.

4. Peptide, dadurch gekennzeichnet, daß sie eines oder mehrere der seroreaktiven Epitope nach den Ansprüchen 1 bis 3 enthalten.

5. Vakzine, dadurch gekennzeichnet, daß sie eines oder mehrere der Peptide nach Anspruch 4 enthalten.

**6.** Diagnostische Zubereitung für die Identifizierung von spezifischen Antikörpern, die gegen die Proteine E1, E6 oder E7 von HPV18 gerichtet sind, dadurch gekennzeichnet, daß sie eines oder mehrere der Peptide nach Anspruch 4 enthält.

**7.** Diagnostische Zubereitung für die Identifizierung von viralen Proteinen in Patientenseren, dadurch gekennzeichnet, daß sie polyklonale oder monoklonale Antikörper mit Spezifität für die Epitope nach Ansprüchen 1 bis 3 und/oder Spezifität für Peptide nach Anspruch 4 enthält.

**Patentansprüche für folgenden Vertragsstaat: ES**

**1.** Verfahren zur Herstellung von Impfstoff gegen HPV, dadurch gekennzeichnet, daß eines oder mehrere der Peptide von HPV 18 mit der Aminosäuresequenz

<div align="center">

TENSPLGERLEVDTELSPRLQEISLNS

</div>

des E1-Proteins

oder mit den Aminosäuresequenzen

    I.     DPTRRPYKLPDLCTELNTSLQDIEITCVYCKT

    II.    MARFEDPTRRPYKL

    III.  AACHKCIDFYSRIRELRHYSDSVYGDTLEKLT

des E6-Proteins

oder mit den Aminosäuresequenzen

    I.     VLHLEPQNEIPVDLLCHEQLSDSEEENDEIDGVNHQHLPARRAEPQRH

    II.    IDGVNHQHLPARR

des E7-Proteins

mit üblichen Adjuvantien und Hilfsstoffen gemischt und für Impfdosen konfektioniert werden.

**2.** Verfahren zur Herstellung eines Diagnostikums, dadurch gekennzeichnet, daß eines oder mehrere der Peptide von HPV 18 mit der Aminosäuresequenz

<div align="center">

TENSPLGERLEVDTELSPRLQEISLNS

</div>

des E1-Proteins

oder mit den Aminosäuresequenzen

I.    DPTRRPYKLPDLCTELNTSLQDIEITCVYCKT

II.   MARFEDPTRRPYKL

III.  AACHKCIDFYSRIRELRHYSDSVYGDTLEKLT

des E6-Proteins

oder mit den Aminosäuresequenzen

I.    VLHLEPQNEIPVDLLCHEQLSDSEEENDEIDGVNHQHLPARRAEPQRH

II.   IDGVNHQHLPARR

des E7-Proteins

an der Oberfläche eines geeigneten Trägermaterials fixiert wird.

3.  Verfahren zur Herstellung eines Diagnostikums, dadurch gekennzeichnet, daß poly- oder monoklonale Antikörper gegen eines oder mehrere der Peptide von HPV 18 mit der Aminosäuresequenz

TENSPLGERLEVDTELSPRLQEISLNS

des E1-Proteins

oder mit den Aminosäuresequenzen

I.    DPTRRPYKLPDLCTELNTSLQDIEITCVYCKT

II.   MARFEDPTRRPYKL

III.  AACHKCIDFYSRIRELRHYSDSVYGDTLEKLT

des E6-Proteins

oder mit den Aminosäuresequenzen

I.    VLHLEPQNEIPVDLLCHEQLSDSEEENDEIDGVNHQHLPARRAEPQRH

II.   IDGVNHQHLPARR

des E7-Proteins

eingesetzt werden.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 257 754  (STANFORD UNIVERSITY)<br>* Das ganze Dokument, insbesondere Seite 2, Zeilen 10-25; Seite 3; Seite 5, Zeile 25 - Seite 6, Zeile 26 *<br>– – – | 1-5 | C 07 K 7/08<br>C 07 K 7/10<br>A 61 K 37/02<br>A 61 K 39/42<br>G 01 N 33/569 |
| A | CHEMICAL ABSTRACTS, Band 107, Nr. 7, 17. August 1987, Seite 191, Zusammenfassung Nr. 53075n, Columbus, Ohio, US; S.T. COLE et al.: "Nucleotide sequence and comparative analysis of the human papillomavirus type 18 genome. Phylogeny of papillomaviruses and repeated structure of the E6 and E7 gene products",<br>& J. MOL. BIOL. 1987, 193(4), 599-608<br>* Zusammenfassung *<br>– – – | 1-5 | |
| A | CHEMICAL ABSTRACTS, Band 106, Nr. 1, 5. Januar 1987, Seite 112, Zusammenfassung Nr. 1115k, Columbus, Ohio, US; G. MATLASHEWSKI et al.: "The expression of human papillomavirus type 18 E6 protein in bacteria and the production of anti-E6 antibodies",<br>& J. GEN. VIROL. 1986, 67(9), 1909-16<br>* Zusammenfassung *<br>– – – – – | 1-5 | |

| RECHERCHIERTE SACHGEBIETE (Int. Cl.5) |
|---|
| C 07 K<br>A 61 K<br>G 01 N |

Der vorliegende Recherchenbericht wurde für alle Patentansprüche erstellt

| Recherchenort | Abschlußdatum der Recherche | Prüfer |
|---|---|---|
| Den Haag | 18 August 91 | MASTURZO P. |